(19) Europäisches Patentamt
European Patent Office
Office européen des brevets

(11) **EP 3 333 176 A1**

(12) **EUROPEAN PATENT APPLICATION**

(43) Date of publication:
**13.06.2018 Bulletin 2018/24**

(51) Int Cl.:
***C07K 7/06*** *(2006.01)* ***C12N 9/12*** *(2006.01)*

(21) Application number: **16203107.4**

(22) Date of filing: **09.12.2016**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD**

(71) Applicant: **UNIVERSITE DE GENEVE**
**1211 Geneva 4 (CH)**

(72) Inventors:
- **RAGUPATHY, Sakthikumar**
  **1205 Geneva (CH)**
- **BORCHARD, Gerrit**
  **1273 Arzier (CH)**

(74) Representative: **reuteler & cie SA**
**Chemin de la Vuarpillière 29**
**1260 Nyon (CH)**

(54) **PEPTIDIC PROTEIN KINASE C INHIBITORS AND USES THEREOF**

(57) The present invention relates to novel peptides, compositions and uses thereof useful in tissue permeabilization, in particular in the context of treatment of cancer prevention and/or treatment or induction of an immune response, in particular *via* mucosal vaccination.

EP 3 333 176 A1

## Description

### Field of the Invention

The present invention relates to new inhibitors of protein kinase C zeta type and their use as tissue permeabilizing agents, in particular in the context of cancer treatment.

### Background of the Invention

Tight junctions (TJ) are complex structures between adjacent epithelial or endothelial cells that regulate passage of ions or molecules through the paracellular space. TJ also determine cell differentiation by giving a clear distinction between the apical and basolateral side. TJ are composed of different segments of proteins namely the transmembrane proteins (claudins, occludin, junctional adhesion molecule (JAM), etc.), and the cytoplasmic scaffolding proteins (ZO-1 (zonula occludens-1), cingulin, afadin, MAGI1 (membrane-associated guanylate kinase), etc.). The cytoskeletal proteins of TJ are actin and microtubules (Van Itallie et al., 2014, Semin. Cell Dev. Biol. 36:157-165).

Protein kinase C (PKC) is a family of serine/threonine kinases that contain a regulatory domain and a catalytic domain. PKCs are implicated in several cellular functions. The PKC isoforms are classified as conventional (α, β1, β2, γ), novel (δ, ε, η, μ, θ), and atypical (ζ, ι/λ) isoforms. Unlike, conventional and novel isoforms, atypical PKC isoforms do not possess a C1 domain (phorbol esters/diacylglycerol binding domain), which is responsible for membrane localization of other PKC isoforms. Peptidic inhibitors of protein kinase C isotype zeta (ζ) have been developed and described as being effective against a wide spectrum of tumors, hyperproliferative disorders such as psoriasis and viral infections such as HIV (WO 93/20101).

Several PKC isoforms have been implicated in the regulation of TJ. Atypical protein PKC zeta is necessary for the assembly of TJ proteins and atypical PKC has been shown to be involved in cell polarity (Steinberg, 2008, Physiol. Rev., 88: 1341-1378; Hirai et al., 2003, J. Biochem., 133: 1-7). PKC zeta and PKC iota share homologically identical amino acid sequences of 72%. This includes a highly conserved pseudosubstrate region (Selbie et al., 1993, J. Biol. Chem., 268: 24296-24302). Pseudosubstrate (PS) region or PS prototope is the sequence present in the regulatory domain responsible for keeping protein kinase in inactive cytoplasmic form by blocking the substrate-binding site present in its kinase domain and corresponds to PKC zeta amino acid sequence 113-126 (House et al., 1987, Science, 238: 1726-1728).

Recent advancements in the knowledge about the molecular architecture of TJ have led to the development of tight junction modulating agents. Epithelial junction openers are tight junction modulating agents that alleviate poor drug absorption, which is a central reason for the failure of oral drug candidates in clinical development (Kennedy, 1997, Drug Discovery Today, 2: 436-444; Lipinski, 2000, J. Pharmacol. Toxicol. Methods, 44: 235-249). The TJ modulating agents that are used as absorption enhancers suffer from a narrow therapeutic window and unspecific mode of action. Toxicity and irreversible opening of TJ is a major reason for the failure of these agents (Deli, 2009, Biochim. Biophys. Acta, 1788: 892-910; Yamamoto et al., 1996, J. Pharm. Pharmacol., 48:1285-1289; Swenson et al., 1994, Pharm. Res., 11: 1132-1142). For example, a small recombinant adenovirus serotype 3-derived protein, termed junction opener 1 (JO-1), which binds to the epithelial junction protein desmoglein 2 (DSG2) was developed and has been shown to increase drug permeability to tumors, in particular to monoclonal antibodies (mAb) used to treat solid tumors. Unfortunately, it has also been shown to cause immunogenicity (Beyer et al., 2011, Cancer Res., 71: 7080-7090).

Carcinomas (including all the subtypes) are malignant transformations of epithelial cells, which account for about 80% of cancer cases. Epithelial tumors are tightly connected by intercellular junctions that restrict penetration through the tumor especially of drugs of a size range of above 500 Da (Lipinski et al., 2001, Adv. Drug Deliv. Rev., 46: 3-26; Lavin et al., 2007, J. Exp. Biol., 210: 2754-2764). Many receptors targeted by antitumor drugs are found hidden/submerged between the tight junctions of the tumor cells that are inaccessible for the antitumor drugs. This is considered one of the important reasons for drug resistance and hence tumor recurrence (Beyer et al., 2011, Cancer Res., 71: 7080-7090). Therefore, in most cases, anti-tumor drugs are inefficacious because of target accessibility issue and not because of lack of drug activity.

Further, mucosal tissues act as a port of entry to various pathogens due to their large surface area of about 400 $m^2$. The immunological component of the mucosal surface, called mucosa-associated lymphoid tissues (MALT), initiates the immune response to an antigen, which then diffuses to *lamina propria* regions. Inducing mucosal immunization is currently the object of extensive research and mucosal vaccination involving the administration of vaccines at one or more mucosal sites for inducing immune responses at the mucosal site of administration, other mucosal sites, and/or systemically is currently extensively investigated. Mucosal vaccination offers several advantages namely a) ease of administration b) stimulation of immunoglobulin A expression, which inhibits adhesion and invasion of microbes. However, mucosal vaccine delivery is hindered by the presence of intercellular TJ that restrict the passage of macromolecules (Borchard et al., 2012, Chitosan-Based Systems for Biopharmaceuticals: Delivery, Targeting and Polymer Therapeutics,

John Wiley & Sons, Ltd, Chapter 12 (Chitosan-based delivery systems for mucosal vaccination), 211-224*).* Therefore, there is a need to develop new TJ modulating agents that are safe, reversible, effective and ideally with a known mode of action.

## Summary of the Invention

The present invention is directed to the finding of novel peptides, which once inside a cell, act as inhibitors of protein kinase C zeta type (PKCζ), which unexpectedly induce a transient redistribution of occludin and ZO-1 inside the intracellular compartment and a transient opening of the tight junctions. This property of the peptides of the invention could be advantageously used for inducing transient tissue permeabilization, in particular for enhancing penetration of large therapeutic molecules such as antibodies or macromolecules used in mucosal vaccination. The tissue permeabilization properties may also be used for the transmucosal delivery of high molecular weight drugs, e.g., peptide and protein drugs such as insulin, avoiding parenteral administration of such drugs. Further, the transiently induced tissue permeabilization would be beneficial in the case of treatments where therapeutic agents are particularly toxic and need to get access to the basolateral side of the target cells, for example in the case of antitumor drugs.

Peptides of the invention were found to significantly increase the efficacy of anti-cancer drugs by increasing the drug penetration into tissues and to significantly improve the immune response to a mucosal vaccine when co-delivered for example as an adjuvant.

It is an object of the invention to provide new inhibitors of PKCζ with low toxicity, ability of inducing efficient and transient tissue permeabilization useful for pharmaceutical use.

A first aspect of the invention provides a compound of Formula (I)/SEQ ID NO.: 1, as well as pharmaceutically acceptable salts and pharmaceutically active variants thereof.

Another aspect of the invention relates to a pharmaceutical composition comprising at least one compound according to the invention.

Another aspect of the invention resides in a compound according to the invention for use in an anti-carcinoma treatment in the prevention and/or treatment of carcinoma.

Another aspect of the invention resides in a compound according to the invention for use in mucosal vaccination.

Another aspect of the invention resides in a use of a compound according to the invention for the preparation of a pharmaceutical composition, in particular a vaccine composition.

Another aspect of the invention is a method for preventing and/or treating a subject suffering from a carcinoma cancer, comprising administering a compound according to the invention or a pharmaceutical formulation thereof in combination with an anti-carcinoma treatment in a subject in need thereof

Another aspect of the invention is a method for inducing immunity comprising administering a mucosal vaccine in combination with a compound according to the invention.

Further objects and advantageous aspects of the invention will be apparent from the claims and/or from the following detailed description of embodiments of the invention with reference to the annexed drawings.

## Brief description of the drawings

**Figure 1** shows the chemical structure of a peptide of the invention according to Formula (I) wherein Z is a myristoyl group, Z1 and Z2 are absent, $Xaa_4$ is Ala, $Xaa_5$ and $Xaa_8$ are Arg and $Xaa_6$ is Trp leading to peptide P1 of SEQ ID NO: 2.

**Figure 2** shows the permeabilization of fluorescein conjugated dextran (4 kDa) as measured by cumulative release induced by peptide of the invention P1 compared to comparative peptides C1 and C2 across nasal (**A**) and bronchial **(B)** epithelial monolayers over a period of 150 minutes as described in Example 2. Values are mean ±S.D. (n=4). The experiments were repeated at least twice. **P* < 0.05, ***P* < 0.01, ****P* < 0.001, *****P* < 0.0001. The data were analyzed for statistical significance using multiple t-test comparisons.

**Figure 3** shows the permeabilization of fluorescein-conjugated dextran (150 kDa) as measured by cumulative release across nasal (**A**) and bronchial **(B)** epithelial monolayers in presence of peptide P1 as compared to control over a period of 150 minutes as described in Example 2.

**Figure 4** shows the permeabilization of fluorescein-conjugated dextran (4 kDa) as measured by cumulative release across nasal (**A**) and bronchial **(B)** epithelial monolayers over a period of 150 minutes in presence of peptide P1 as compared to comparative peptide C3 and control over a period of 150 minutes as described in Example 3.

**Figure 5** shows the apparent permeability $P_{app}$ of fluorescein conjugated dextran (4 kDa) as a function of TJ opening at different time points for peptide of the invention P1 compared to peptides C1 and C2 tested in nasal (**A**) and bronchial **(B)** epithelial monolayers over a period of 150 minutes as described in Example 4.

**Figure 6** shows the apparent permeability ($P_{app}$) of FD (150 kDa) as a function of TJ opening at different time points

for P1 tested in nasal (**A**) and bronchial **(B)** epithelial monolayers over a period of 150 minutes. Values are mean ±S.D. Number of experiments, analysis and P values in figures 3 to 6 are as in Figure 2.

**Figure 7** shows the cell viability measured as described in Example 5 after 24h exposure in presence of peptide of the invention P1 as compared to comparative peptides C1 and C2 and SDS at 2%. Values are mean ± S.D. (n=4). **** p<0.0001. The data were analyzed using one-way analysis of variance (ANOVA).

**Figure 8** shows immunofluorescence labelling for TJ proteins occludin-1 and ZO-1 in primary human bronchial epithelial cell monolayers (**A**) and primary human nasal epithelial cell monolayers **(B)** treated with peptide of the invention P1 or comparative peptides C1 or C2 as described in Example 6. All the experiments were performed three times with the number of replicates of n=5. Scale bar indicates 10 μm.

**Figure 9** compares the effect on tumor size of repeated exposures of the OCA_EGFR19del cell cultures with 1 μM of peptide P1 or DMSO (control) as described in Example 7.

**Figure 10** shows the effect on tumor size of a combination of gefitinib (basal administration, 1 μM (**A**) or 5 μM **(B)**) and P1 (apical administration, 1 μM) and of a combination of gefitinib (apical administration, 1 μM) and P1 (apical administration, 1 μM) **(C)** on OCA_EGFR19del cultures, as described in Example 7 compared to control (DMSO) and gefitinib alone. For each time point, data are means ± SEM (n=3). *- statistically significant difference between combination and single treatment (2-way ANOVA).

**Figure 11** shows antigen-specific serum IgG (**A**) or IgG1 **(B)** total titers of seropositive mice (n=5) 1 week after the boost vaccination as described in Example 8. Serum samples of control group were collected from un-immunized mice (n = 3). Error bars indicated 95% confidence intervals. *P =0.0079.

## Detailed Description of the invention

The term "cell penetrating moiety" refers to a peptidic or non-peptidic moiety with the ability to translocate across lipid bilayers (e.g. cell membranes). When a cell penetrating moiety is conjugated to another molecule (cargo) it aids or enhances the efficient transit of a said cargo molecule across lipid bilayers (e.g. cell membranes) into cells or tissue and also across blood-brain barrier in other words a cell penetrating moiety acts as a transmembrane carrier.

The cell penetrating moiety can be a fatty acid moiety and it can be covalently linked to a peptide backbone for example by acylation, for example by N-myristoylation or palmitoylation. Examples of fatty acids that can be used as cell penetrating moiety according to the invention include caprylic acid (octanoic acid; C8:0), capric acid (decanoic acid; C10:0), lauric acid (dodecanoic acid; C12:0), myristic acid (tetradecanoic acid; C14:0), palmitic acid (hexadecanoic acid, C16:0), stearic acid (octadecanoic acid, C18:0), arachidic acid (icosanoic acid, C20:0), behenic acid (docosanoic acid, C22:0), lignoceric acid (tetracosanoic acid, C24:0), cerotic acid (hexacosanoic acid).

Alternatively, a cell penetrating moiety in the context of the invention can be a lipidic cell penetrating moiety conjugated to another cell penetrating moiety such as in lipidic cell penetrating nanoparticles or cationic liposomes, for example as in LipofectAMINE® formulation (Thermo Fisher Scientific, Waltham, MA, USA), myristoyl-Arg7, stearyl-Arg8, cholesteryl-Arg9, stearyl-TP10 (named PepFect3), stearyl-$(Arg\text{-}Ahx\text{-}Arg)_4$, C12R9, C12dR9, C12dR9-1, C12dR9-2, C14R11, C14dR11 (*Lee et al., 2013, supra; Di Pisa et al., 2015, supra)* or in a vector comprising palmitoyl chain and arginine residues *(*Bonnet et al., 2001, J. Med. Chem., 44: 468-471*).*

Alternatively, a cell penetrating moiety can be a peptidic sequence derived from a natural protein or a chimeric peptide formed by the fusion of two natural sequences or a synthetic peptide which is rationally designed. Examples of a peptidic cell penetrating moiety include, but are not limited to, TAT (trans-activator of transcription of HIV), *Drosophila* homeotic protein antennapedia (ANTp, penetratin), W/R, NLS (nuclear localization signal), $AlkCWK_{18}$, $DiCWK_{18}$, transportan, DipaLytic, $K_{16}RGD$, P1, P2, P3, P3a, P9.3, Plae, Kplae, cKplae, MGP, HA2, $LARL4_6$, (LARL)n, Hel-11-7, KK, KWK, RWR, loligomer, Herpes virus VP22, SCWKn, RGD, 8-Lysine, MPG, pVEC, ARF (1-22), BPrPp (1-28), VT5, MAP, SG3, Pep-7, FGF (fibroblast growth factor), stapled peptides, prenylated peptides, pepducins, Pep-1, polyarginines (9-Arginine, 8-Arginine, 6-Arginine), $R_6W_3$, TP10, arginine-rich peptides like $(Arg\text{-}X\text{-}Arg)_n$ peptides (where X is a generic carbon chain spacer), proline-rich peptides, *(*Schwartz et al., 2000, Curr. Opin. Mol. Ther., 2(2): 162-7*;* Lee et al., 2013, Methods Mol Biol., 991:281-92*;* Bechara et al., 2013, FEBS Lett., 587(12): 1693-1702*; Di* Pisa et al., 2015, J. Pept. Sci., 21(5): 356-369*;* Guo et al., 2016, Biomed. Rep., 4(5):528-534*)*. According to a particular aspect, a cell penetrating moiety is as described in Svensen et al., 2012, Trends in Pharmacological Sciences, 33(4): 186-192*.*

According to one aspect, the cell penetrating moiety can be conjugated to the rest of the backbone of the peptide of the invention through thiazolidine, thioether, disulfide, or hydrazone linkages using known ligation protocols *(Bonnet et al., 2001, supra).* Alternatively, a cell penetrating moiety in the context of the invention can comprise a homing peptide (HP) sequence for targeting specifically the tight junctions in certain cells, for example cancer cells in case of cancer homing peptides. For example, a cell penetrating moiety according to the invention can comprise a homing peptide (HP) sequence conjugated to a cell penetrating moiety or a cell-penetrating homing peptide (CPHP), for example as described in Svensen et al., 2012, Trends in Pharmacological Sciences, 33(4): 186-192*.* According to another aspect, a peptide according to the invention can be further conjugated to a homing peptide, for example at its C-terminus.

A "homing peptide" or HP refers to a peptide that has no inherent internalization properties and only delivers its cargo to specific cell-surface receptors, other HP have cell penetrating properties *per se.*

The term "myristoylation" refers to the conjugation of a myristoyl group through an amide bond to an amino acid of the peptide of the invention, in particular the alpha-amino group of the N-terminal residue.

The term "therapeutic molecule" refers to a molecule used in a treatment or prevention of a disease. Examples of therapeutic molecules in the context of the invention include, but are not limited to, molecules used in prophylactic vaccines (used in a process of acquiring immunity to a particular disease or pathogen), molecules used in therapeutic vaccines (e.g., vaccines for cancer treatment or vaccines to induce tolerance against an allergen), therapeutic antibodies (e.g., antibodies for cancer treatment), low molecular weight drugs (e.g., cytotoxic drugs or enzyme inhibitors used in cancer treatment) and anesthetic agents.

The term "protein kinase C zeta type" or "PKCζ" refers to a type of protein kinase C isoform. The term "tight junctions" abbreviated "TJ" refers to complex structures between adjacent epithelial or endothelial cells that regulate passage of ions or molecules through the paracellular space. TJ are composed of different segments of proteins namely transmembrane proteins such as claudins, occludin, junctional adhesion molecule (JAM), etc., and cytoplasmic scaffolding proteins such as ZO-1, cingulin, afadin, membrane associated guanylate kinase (MAGI1), etc. The effect of peptides of the invention on tight junction can be monitored through i) measurement of transepithelial electrical resistance (TEER); ii) determination of the apparent permeability (Papp) of paracellular markers (e.g., fluorescein-dextrans); iii) fluorescent immunostaining of TJ proteins followed by imaging; iv) determination of mRNA and protein expression of TJ proteins.

The term "carcinoma" as defined herewith is a disease involving malignant transformations of epithelial cells (including all the subtypes of carcinoma). Term "carcinomas" designates diseases exemplified by, but not limited to breast, prostate, lung, pancreas, esophageal, hepatocellular, ovarian, colorectal and head and neck cancers, and other solid tumors.

The term "mucosal vaccine" as defined herewith refers to a vaccine that is administered at one or more mucosal sites leading to induction of immune responses at the mucosal site of administration, other mucosal sites, and/or systemically. The mucosal tissues comprise nasal, oral, intestinal, pulmonary, ocular, rectal and vaginal tissue.

The term "efficacy" of a treatment according to the invention can be measured based on changes in the course of disease in response to a use or a method according to the invention. For example, the efficacy of a treatment according to the invention can be measured by its impact on signs or symptoms of illness. A response is achieved when the subject experiences partial or total alleviation, or reduction of unwanted symptoms of illness. According to a particular embodiment, the efficacy can be measured through the assessment of an increase of the effect of a therapeutic molecule used in the combination with the compound of the invention as compared to the effects of the same molecule used alone. For example, the efficacy of an anti-cancer treatment according to the invention can be monitored by following the effect on the tumor size or by the improvement of survival among the patient group thus treated.

The term "efficacy" of a treatment according to the invention can be measured based on a decrease in the treatment side effects compared to a treatment administered without the peptides of the invention.

The term "efficacy" of a vaccine according to the invention can be measured based on changes in immune system response. For example, the efficacy of a vaccination according to the invention can be measured by its impact on the acquired immunity to a particular disease/pathogen. For example, a response to a vaccination is achieved when the subject acquires specialized, systemic cells and processes that eliminate or prevent pathogen growth. The invention may also be used to increase the efficacy of an allergy vaccine (induction of tolerance) by increasing the mucosal (e.g., oral or nasal) penetration of an allergen, e.g., a recombinant allergen.

As used herein, "treatment" and "treating" and the like generally mean obtaining a desired pharmacological and physiological effect. The effect may be prophylactic in terms of preventing or partially preventing a disease, symptom or condition thereof and/or may be therapeutic in terms of a partial or complete cure of a disease, condition, symptom or adverse effect attributed to the disease.

The term "permeabilization" as used herein refers to a process of making a membrane permeable to an agent present on one side of the membrane. In the context of the invention, permeabilization achieved by the compounds of the invention enhances the penetration of molecules across epithelial layers. The ability of compounds of invention to increase tissue permeability to some agents can be tested in known assays such as those described below. The term "subject" as used herein refers to mammals. For examples, mammals contemplated by the present invention include human, primates, domesticated animals such as cattle, sheep, pigs, horses, laboratory rodents and the like.

In the context of the invention, a non-polar amino acid can be selected from Gly, Ala, Val, Leu, Ile, Met, Trp, Phe and Pro or a conservative substitution thereof.

In the context of the invention, a positively charged amino acid is selected from Arg, Lys or His or a conservative substitution thereof.

For example, a "conservative amino acid substitution" may involve a substitution of a native amino acid residue with a non-native residue such that there is little or no effect on the polarity or charge of the amino acid residue at that

position. Desired amino acid substitutions can be determined by those skilled in the art at the time such substitutions are desired. The term "variant" also includes a peptide or polypeptide substantially homologous to the referenced peptide sequence, but which has an amino acid sequence different from that of the referenced sequence because one or more amino acids have been chemically modified or substituted by amino acids analogs. For example non-natural residues can be introduced to enhance the pharmacological properties of peptide-based therapeutics (Geurink et al., 2013, J. Med. Chem., 56, 1262*;* Rand et al., 2012, Med. Chem. Commun, 3, 1282*).*

According to another particular embodiment, the peptides of the invention can be optionally amidated at the C-terminus.

The term "pharmaceutical formulation" refers to preparations which are in such a form as to permit biological activity of the active ingredient(s) to be unequivocally effective and which contain no additional component which would be toxic to subjects to which the said formulation would be administered.

***Compounds of the invention***

According to one aspect, is provided a peptide of 5 to 10 amino acids in total of the following Formula (I):

Z- Z1-$Xaa_4$ $Xaa_5$ R $Xaa_7$ $Xaa_8$ -Z2 **(I)**

wherein **Z** is a cell penetrating moiety;
**Z1** is an optional peptidic moiety of 1 to 3 amino acids of formula (II):

Xaa1 Xaa2 Xaa3 **(II)**

wherein Xaa1 and $Xaa_2$ can be present or absent and, when present, Xaa1 and $Xaa_2$ are independently a positively charged amino acid, more particularly Arg and $Xaa_3$ is a non-polar amino acid, in particular Gly;
$\mathbf{Xaa_4}$ is an amino acid selected from Ala, Ser and Val, more particularly Ala;
**R** is Arginine;
$\mathbf{Xaa_5}$ and $\mathbf{Xaa_8}$ are independently a positively charged amino acid, more particularly Arg; $\mathbf{Xaa_7}$ is a non-polar amino acid, more particularly Trp;
**Z2** is an optional peptidic moiety of 1 to 2 amino acids of formula (III):

$Xaa_9$ $Xaa_{10}$ **(III)**

wherein $Xaa_9$ is a positively charged amino acid, more particularly Lys and $Xaa_{10}$ can be present or absent and, when present, $Xaa_{10}$ is a non-polar amino acid, in particular Leu. According to a particular embodiment, is provided a peptide of Formula (I), wherein the said cell penetrating moiety Z is covalently attached to the N-terminus of the peptide.

According to another particular embodiment, is provided a peptide of 5 to 10 amino acids in total of Formula (I) which can be represented by the amino acid consensus sequence of SEQ ID NO: 1.

According to another further particular aspect, is provided a peptide of Formula (I) wherein Z1 is absent.

According to another further particular aspect, is provided a peptide of Formula (I) wherein Z2 is absent.

According to another further particular aspect, is provided a peptide of Formula (I) wherein Z1 and Z2 are absent.

According to another further particular aspect, is provided a peptide of Formula (I) wherein $Xaa_4$ is Ala.

According to another further particular aspect, is provided a peptide of Formula (I) wherein $Xaa_5$ is Arg.

According to another further particular aspect, is provided a peptide of Formula (I) wherein $Xaa_5$ is Lys.

According to another further particular aspect, is provided a peptide of Formula (I) wherein $Xaa_8$ is Arg.

According to another further particular aspect, is provided a peptide of Formula (I) wherein $Xaa_5$ and $Xaa_8$ are Arg.

According to another further particular aspect, is provided a peptide of Formula (I) wherein $Xaa_5$ is Lys and $Xaa_8$ is Arg.

According to another further particular aspect, is provided a peptide of Formula (I) wherein $Xaa_6$ is Trp.

According to another further embodiment, is provided a peptide of the invention of the following Formula (Ia):

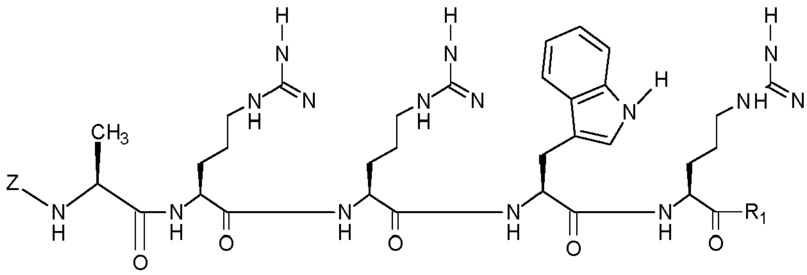

**(Ia)**

Wherein Z is as described herein and $R_1$ is selected from OH and an amino group, such as $NH_2$.

According to a further particular aspect, the said cell penetrating moiety Z is a fatty acid moiety.

According to another further particular aspect, the fatty acid moiety is a myristoyl group.

In another further particular embodiment is provided a peptide of the invention of SEQ ID NO: 2 (Peptide P1).

According to another further embodiment, is provided a peptide of the invention of SEQ ID NO: 3.

According to one embodiment, compounds of the invention may be prepared by synthetic methods, in particular by solid phase peptide synthetic. According to an embodiment, non-commercial cell penetrating moieties can be first prepared separately according to standard methods before grafting.

According to a particular embodiment, compounds of the invention are inhibitors of protein kinase C zeta type (PKCζ).

According to a particular embodiment, compounds of the invention are transient tight junction opening agents.

***Compositions***

Pharmaceutical compositions of the invention can contain one or more compound according to the invention and a pharmaceutically acceptable carrier, diluent or excipient thereof. According to a particular aspect, compositions further comprise a compound useful in a treatment of a medical disorder or in vaccine.

According to a particular aspect, compositions of the invention are anticancer compositions. According to another particular aspect, compositions of the invention are vaccine compositions.

Compositions of this invention may further comprise at least one agent useful in a treatment of a carcinoma.

According to a particular aspect, is provided a pharmaceutical composition according to the invention wherein the agent useful in a treatment of a carcinoma is selected from a protein (e.g., an antibody), a kinase, Designed Ankyrin Repeat Proteins (DARPins), small molecules or any other active substance suitable/approved for cancer treatment.

According to a further particular embodiment, is provided a pharmaceutical composition according to the invention comprising at least one peptide of the invention and at least gefitinib.

Compositions of this invention may further comprise at least one agent useful in vaccination, in particular mucosal vaccination such as recombinant B subunit of cholera toxin and inactivated vibrio cholerae O1 (Inaba and Ogawa serotype), killed whole cells of *V. cholerae* O1 and *V. cholerae* 0139, live attenuated rotavirus type p1a (8), g1-g4 or type rix 4414, attenuated live strain of Salmonella typhi Ty21a, live attenuated influenza virus, live attenuated, monovalent or pentavalent rotaviruses, live attenuated trivalent, bivalent and monovalent polioviruses, live attenuated *S. typhi* bacteria, inactivated *V. cholera* O1 classical and E1 Tor biotypes with or without cholera toxin B subunit (CTB) *(*Mevyn et al., 2014, Human Vaccines & immunotherapeutics, 10(8): 2175-2187*;* Sae-Hae et al., 2014, Experimental & Molecular Medicine, 46: e85*).*

Compositions of this invention may further comprise one or more pharmaceutically acceptable additional ingredient(s) such as alum, stabilizers, antimicrobial agents, buffers, coloring agents, flavoring agents, adjuvants, and the like.

Compositions of this invention may also be formulated for parenteral administration including, but not limited to, by injection or continuous infusion. Formulations for injection may be in the form of suspensions, solutions, or emulsions in oily or aqueous vehicles, and may contain formulation agents including, but not limited to, suspending, stabilizing, and dispersing agents. The composition may also be provided in a powder form for reconstitution with a suitable vehicle including, but not limited to, sterile, pyrogen-free water. Compositions of this invention may be formulated for inhalation, which may be in a form including, but not limited to, a solution, suspension, or emulsion that may be administered as a dry powder or in the form of an aerosol using a propellant, such as dichlorodifluoromethane or trichlorofluoromethane.

According to a particular embodiment, compositions according to the invention are for intra-tumoral injection.

According to a particular embodiment, compositions according to the invention are for mucosal surface delivery.

In another particular aspect, compositions according to the invention are adapted for delivery by single or multiple administrations.

Alternatively, compositions of this invention may also be formulated as an aerosolable solution or an inhalable pharmaceutically acceptable composition. In such a formulation, the compound according to the invention is prepared for example as an inhalable dry powder or as an aerosolable solution.

According to a particular embodiment, compositions of the invention are veterinary compositions.

Further materials as well as formulation processing techniques and the like are set out in Part 5 of Remington's "The Science and Practice of Pharmacy", 22nd Edition, 2012, University of the Sciences in Philadelphia, Lippincott Williams & Wilkins, which is incorporated herein by reference.

The invention provides peptides of the invention, compositions thereof and methods using the same useful in the treatment of a medical disorder, in particular carcinoma or in a vaccination process.

***Mode of administration***

Compositions of this invention may be administered or delivered in any manner including, but not limited to, orally, parenterally, sublingually, transdermally, transmucosally, topically, via inhalation, via buccal or intranasal administration, or combinations thereof. Parenteral administration includes, but is not limited to, intra-tumour, intra-intravenous, intra-arterial, intra-peritoneal, subcutaneous and intramuscular.

In another particular embodiment, a compound according to the invention is administered systemically by injection.

In another particular embodiment, a compound according to the invention is administered by inhalation.

In another particular embodiment, a compound according to the invention is administered transmucosally.

In another particular embodiment, a compound according to the invention is administered intra-tumorally.

In a specific embodiment, the method according to the invention is a method of administering a compound according to the invention to the tumour in the lungs of a subject, comprising bronchoscopy guided intra-tumour injection of a compound of the invention or a composition thereof.

The dosage administered, as single or multiple doses, to an individual will vary depending upon a variety of factors, including pharmacokinetic properties, subject conditions and characteristics (sex, age, body weight, health, and size), extent of symptoms, concurrent treatments, frequency of treatment and the effect desired.

***Combination***

According to one aspect, compounds of the invention are to be administered in combination with at least one therapeutic molecule useful in the prevention and/or treatment of a disease. According to one aspect, compounds of the invention are to be administered in combination with at least one therapeutic molecule useful in the prevention and/or treatment of a carcinoma.

According to one aspect, compounds of the invention are to be administered in combination with therapeutic molecules useful for vaccination, in particular mucosal vaccination.

The invention encompasses the administration of a compound of the invention wherein the compound is administered to a subject prior to, simultaneously or sequentially with a therapeutic regimen or at least one co-agent. The compound according to the invention that is administered simultaneously with said at least one co-agent can be administered in the same or different compositions and in the same or different routes of administration.

According to one aspect, compounds of the invention can be administered simultaneously, optionally in the same composition, with at least one therapeutic molecule useful for the treatment of a lung cancer.

According to a further particular aspect, compounds of the invention can be administered intratumorally through guided bronchoscopy.

The compound according to the invention can be administered simultaneously, optionally in the same composition, with at least one vaccine composition.

***Patients***

In an embodiment, subjects according to the invention are suffering from or at risk of suffering from a carcinoma.

In a further embodiment, subjects according to the invention are suffering from or at risk of suffering from a cancer selected from a breast, prostate, lung, pancreas, esophageal, hepatocellular, ovarian, colorectal and head and neck cancer, and other solid tumors.

In a further embodiment, subjects according to the invention are suffering from or at risk of suffering from a lung cancer.

In another embodiment, subjects according to the invention are subject to a mucosal vaccination, such as for

example vaccination against influenza virus, rotavirus, *Vibrio Cholerae, Salmonella thyphi* or poliovirus infections.

### *Use according to the invention*

The compounds according to the invention are useful in enhancing the effects of therapeutic molecules used in the prevention and/or treatment of any diseases, in particular those used in the prevention and/or treatment of a carcinoma or in vaccination.

According to another aspect, compounds according to the invention can be used in view of the delivery of agents through the blood-brain barrier (BBB), across the skin, or for improving diffusion of anesthetic agents through a tissue in view of improving local anesthesia.

According to a particular aspect, the peptides of the invention present various advantages over known PKCζ pseudosubstrates, in particular Myr-SIYRRGARRWRKL (comparative peptide C1 of SEQ ID NO: 4), which was used as a tool for investigating the role of PKCζ in tight junction regulation and described as being able to disrupt tight junctions in mouse *ileum* (Jain et al., 2011, Biochem J., 437(2), 289-299*)* but was not suggested for use as a permeabilizing agent and even less in combination with therapeutic or vaccine macromolecules for enhancing their efficacy. In particular, peptides of the invention were unexpectedly found not only to be less toxic at higher concentrations than this pseudosubstrate but also to exhibit advantageous transient membrane permeabilization ability.

References cited herein are hereby incorporated by reference in their entirety. The present invention is not to be limited in scope by the specific embodiments described herein, which are intended as single illustrations of individual aspects of the invention, and functionally equivalent methods and components are within the scope of the invention. Indeed, various modifications of the invention, in addition to those shown and described herein will become apparent to those skilled in the art from the foregoing description. Such modifications are intended to fall within the scope of the appended claims. The invention having been described, the following examples are presented by way of illustration, and not limitation.

## EXAMPLES

The following abbreviations refer respectively to the definitions below:

**BSA** (bovine serum albumin); **C1** (comparative peptide 1); **C2** (comparative peptide 2); **C3** (comparative peptide 3); **Caco-2** (human intestinal epithelial cells); **CTRL** (control); **DIEA** (N,N-Diisopropylethylamine); **DCM** (dichloromethane); **DMF** (N,N-dimethylformamide); **EDT** (ethanedithiol); **EGFR** (epidermal growth factor receptor); **FD** (fluorescein conjugated dextran); **Fmoc** (9-Fluorenylmethyloxycarbonyl); **HBTU** (2-(1H-benzotriazol-1-yl)-1,1,3,3-tetramethyluronium hexafluorophosphate); **HOBT** (hydroxybenzotriazole); **i.n**. (intranasal); **myr** (myristoyl group); **NSCLC** (non-small cell lung cancer); **OCA_EGFR19del** (cell cultures with tumors carrying the EGFR ex19:del mutation); **Ova** (ovalbumin); **P1** (peptide 1); **$P_{app}$** (apparent permeability); **PBS** (phosphate buffered saline); **PKCζ** (protein kinase C zeta type); **PS** (Pseudosubstrate); **TEER** (trans-epithelial electrical resistance); **TIS** (triisopropylsilane); **TFA** (trifluoroacetic acid); **TJ** (tight junctions); **ZO-1** (zonula occludens-1).

### Example 1: Synthesis of compounds according to the invention

Compounds of the invention are prepared by solid phase peptide synthesis. As an illustration, the steps of the synthesis of Peptide 1 (P1, SEQ ID NO: 2, **Figure 1**) are provided below:

*Step 1*- The reaction vessel was washed with dichloromethane (DCM) and bottom blown with nitrogen and then drained completely.

*Step 2*- Resin swelling: 2-Chlorotrityl Chloride Resin was weighed in the reaction vessel, the resin was then swollen with dimethylformamide (DMF; 15ml/g) for 30 min.

*Step 3*- Coupling of the first amino acid from the C-terminus of the peptide: 1.6 g of Fmoc-L-Arg(Pbf)-OH were weighted in a test tube and Fmoc (9-Fluorenylmethyloxycarbonyl)-amino acids were dissolved in DMF/DCM (Sigma-Aldrich) (1:1) (15ml/g). The solution was transferred into the reaction vessel described above, 10 times DIEA (N,N-Diisopropylethylamine) was added and mixed for 30 min at room temperature with nitrogen. *Step* 4- Blocking the active site of the resin: 5 mL of methanol was added into the reaction vessel and bottom blown for 10 min. The reaction vessel was drained and washed with DMF (3x), DCM (3x) and DMF (3x).

*Step* 5- Deprotection: The reaction vessel was drained and then 20% piperidine (15 ml/g) was added to remove the Fmoc protective group. The mixture was bottom blown for 10 min x1 and 5 min x1. The reaction vessel was then washed with DMF (3x), DCM (3x), DMF (3x). *Step* 6- Coupling Monitoring: A sample of resin was taken and 2 drops of 25% ninhydrin-alcohol solution and 1 drop of 20% phenolic-alcohol solution, and then 1 drop of pyridine were

added, the sample was next heated in 105°C for 5 min., the colour change into deep blue indicated a positive reaction and the absence of colour change is indicative of an absence of reaction.

*Step* 7- Condensation: 3 times excess of protected amino acid, 5g of HBTU (2-(1H-benzotriazol-1-yl)-1,1,3,3-tetramethyluronium hexafluorophosphate), HOBT (hydroxy benzotriazole) (1 g) and DIEA (2 ml) were added in DMF to be dissolved and then DCM (15 ml/g) was added and the mixture was let to react for 1 hour.

*Step* 8- Washing: The reaction vessel was washed with DCM (15 ml/g) and DMF (15 ml/g) alternately 3 times.

*Step 9-* Monitoring: as in step 6.

*Step 10-* Coupling the remaining amino acids: Steps 5-9 were repeated to couple the other amino acids.

*Step 11-* Linking myr group on the peptide N-terminus.

*Step* 12- Washing: The resin was washed after the last amino acid (first amino acid from the N-term) coupling and deprotection with the following reagents in turn: 2 times DMF (10 ml/g), 2 times methanol (10 ml/g), 2 times DMF (10 ml/g), 2 times DCM (10 ml/g) and then was draw drying for 10 min.

*Step 13-* Cleavage: Cleavage was performed with the following reagent: TFA 94.5% (trifluoroacetic acid), water 2.5%, EDT 2.5% (ethanedithiol), TIS 1% (triisopropylsilane). The cleavage time was 2 hours.

*Step 14-* Blow drying and wash: The cleavage solution was blow dried with nitrogen gas as far as possible, and washed 6 times with absolute ether and dried in air.

*Step 15-* Purification by HPLC (high-performance liquid chromatography). The purified solution was dried by freeze drying, and the white-powder-form product was obtained.

Purification by HPLC

The crude peptide was dissolved in purified water and purified under the following conditions Dissolve the crude peptide with purified water. Purification condition is below:

Pump A: 0.1% trifluoroacetic acid in 100% water 0.1% TFA-100% water solution
Pump B: 0.1% trifluoroacetic acid in 100% acetonitrile 0.1% TFA-100% ACN solution
Preparation column: Venusi MRC-ODS C18 30 x 250 mm.
Preparative Column: Venusi MRC-ODS C18 30 x 250 mm
Total flow rate: 1.0 ml / min Flow rate: 1.0 ml / min
Loading volume: 3 ml Sampling volume: 3 ml
Detection wavelength: 220 nm Detection wave: 220 nm

Gradient gradient

| Time (min) | A | B |
|---|---|---|
| 05.00 | 90% | 10% |
| 30.00 | 20% | 80% |
| 30.10 | Stop | |

The synthesis of other peptides of the invention can be prepared in a similar manner by using different or additional amino acids to lead to a peptide of SEQ ID NO: 1. The grafting of the cell penetrating moiety can be achieved though standard methods known to the skilled person such as solid phase synthesis in the case of peptidic cell penetrating moieties or as described in the present description.

Compounds of the invention can be also prepared chemoselective ligation synthesis *(Bonnet et al., 2001, supra).*

**Example 2: Effects of peptides of various lengths increase on membrane permeability of macromolecules**

To assess the potential effects of peptides of the invention on the permeabilization of therapeutic molecules, fluorescein-conjugated dextrans (FD) were used to as a model for assessing paracellular drug transport (apical to basolateral) across the epithelial monolayer. To ensure that the integrity of the monolayer was maintained during the course of the experiment, trans-epithelial electrical resistance (TEER) was measured before and after these studies, as described below.

Mucilair™ human primary nasal and bronchial epithelial cells (Epithelix sarl, Geneva, Switzerland) were used *(*Huang et. al., 2013, Toxicol. in Vitro 27: 1151-1156*).*

Before each experiment, the culture medium was removed from each compartment and the monolayer was washed once with 200 μl of saline (0.9%) and once with warm Hanks' Balanced Salt Solution (HBSS) (37°C). In the basolateral compartment, 600 μL of prewarmed HBSS was placed and the cells were returned to the incubator at 37°C

for 30 minutes to equilibrate. After equilibration, the peptides of the invention were applied to the apical side of an epithelial monolayer 5 minutes prior to the addition of FD solution. Peptide 1 according to the invention (**P1** of SEQ ID NO: 2) was tested in comparison with comparative peptides **C1** (SEQ ID NO: 4) and C2 (SEQ ID NO: 5). FD having a molecular weight (MW) of 4 and 150 kDa were used.

All peptides were used at a final concentration of 10 μM. The vehicle was used as a control (CTRL). The FD solution was added to the apical compartment to make a final volume of 200 μl. Samples of 100 μL were taken from the basal compartment of each well every 30 minutes over a period of 150 minutes, with each volume being replaced with equal amount of fresh warm buffer to maintain sink condition. The fluorescence of FD was measured in black 96-well plates using a fluorescence plate reader (BioTek Synergy Mx plate reader, BioTek Instruments GmbH, Lucerne, Switzerland), using excitation and emission wavelengths of 485 and 520 nm, respectively. Cumulative release (ng) corresponds to the actual amount of drug released cumulatively and corresponds to the amount in the suspension medium at any time plus the amount of the drug lost during each sampling.

*Trans-epithelial electrical resistance (TEER):* After addition of 200 μl of culture medium to the apical compartment of the tissue cultures, resistance was measured across cultures with an EVOMX volt-ohm-meter (World Precision Instruments UK, Stevenage) in triplicate for each time point. The TEER values (Ω) were converted normalized by using the following formula: TEER (Ω $cm^2$) = (resistance value (Ω)-100 (Ω))×0.33 ($cm^2$), where 100 Ω is the resistance of the membrane and 0.33 $cm^2$ is the total surface of the epithelium.

Peptide P1 and comparative peptide C1 increased the permeability of the fluorescein conjugated dextran of 4 kDa in a significant manner in both nasal **(Figure 2A)** and bronchial **(Figure 2B)** human primary epithelial cells, while comparative peptide C2 was not differing differently from control. Bronchial epithelial cells were more sensitive when compared to nasal epithelial cells. Furthermore, P1 significantly increases the permeability of FD of a molecular weight of 150 kDa, which is equivalent to the size of an antibody when compared to the control, both in nasal **(Figure 3A)** and bronchial **(Figure 3B)** epithelial cells.

These results support that the peptides of the invention are able to permeabilize the macromolecules to the same extend to a much longer comparative peptide and thus could significantly improve permeability of drugs of a size up to 150 kDa across epithelial monolayers.

**Example 3: Role of the membrane penetrating group**

To assess the role of the membrane penetrating group (myristoyl) in peptides of the invention in the enhancing of the permeability of macromolecules through epithelial cell layers, the permeabilizing capacity of peptide 1 of the invention was compared to a comparative peptide C3 (SEQ ID NO: 6) corresponding to the same peptide without the myristoyl group.

Uptake experiments and TEER were conducted as described in Example 2.

Comparative peptide C3 did not show significant difference in permeabilization ability compared to the negative control in both nasal **(Figure 4A)** and bronchial **(Figure 4B)** human primary epithelial cells, suggesting that a membrane penetrating group, in particular a N-myristoyl group, is necessary for the peptides of the invention to enhance permeabilization.

**Example 4: Paracellular permeability time frame**

To assess the duration of the permeabilization effect of the peptide of the invention, apparent permeability of peptides of various lengths was evaluated as follows. Apparent permeability refers to the amount of released FD at that time point when the sample is collected. In this example, it indirectly signified the gradual closing of tight junction paracellular space as the amount of released FD gradually decreased as seen at the time points of 30 or 60 minutes. The tested Peptides, uptake experiments and TEER were as described in Example 2. *Apparent permeability of FD:* The apparent permeability ($P_{app}$) of FD was calculated using equation 1: $P_{app} = (dQ/dt)/A * C_0$ **(1)**

Where $C_0$ is the initial concentration (ng/ml) of FD in the donor compartment (i.e. where the FD is added, A ($cm^2$) is the surface area of the cell layers (0.33 $cm^2$ for 24 well plate inserts) and dQ/dt is the appearance rate of FD in the receiver compartment B (receiver compartment is the basolateral compartment while the donor compartment is the apical compartment). $C_0$ did not change significantly over the 150 minute flux study.

The effect of peptides P1 and comparative peptide C1 on permeabilization of FD 4 kDa in both nasal and bronchial epithelial cells was reversible as shown by the decrease in $P_{app}$ overtime. The ranking of the degree of reversibility was C2>P1>C1 **(Figure 5),** indicating that the degree of reversibility increased with a decrease in the peptide length. Reversibility of the permeabilization effect was also observed for P1 with the high molecular weight FD of 150 kDa. The $P_{app}$ for FD of 150 kDa showed an immediate increase in the first 30 minutes and steadily decreased therefrom **(Figure 6).**

These results support that the enhancing effect of paracellular permeability of the peptides of the invention is

transcient suggesting that those would be able to induce a transient opening of the tight junctions.

It is hypothesized that since the peptides corresponds to PKC pseudosubstrates, they might be competitive substrates and therefore, the higher the length of pseudosubstrate sequence, the higher is the inhibitory effect on PKC zeta and consequently the higher is the increase in paracellular permeabilization of macromolecules. When the length of the pseudosubstrate sequence is decreased to 5 (P1) from 13 (C1) amino acids, a decrease in paracellular permeability is observed together with an increase in the reversibility of permeabilizing effect. Thus, peptides of the invention, in particular peptide P1 have an optimized length which allows to achieve desirable paracellular permeabilization of macromolecules while at the same time presenting a higher reversibility of this effect, which is desirable for avoiding cytotoxicity (longer times of PKC zeta inhibition potentially will lead to an increase of disassembly of TJ proteins and affects cell proliferation) *(*Suzuki et. al., 2002, J. Cell Sci., 115: 3565-3573*;* Whyte et. al., 2010, J. Cell Sci., 123: 3316-3328*).*

**Example 5: Effect of PKC zeta PS peptides on cell viability**

The assessment of the potential toxicity of the peptides of the invention compared to comparative peptides was performed on a cell viability assay as follows.

Viability of Caco-2 cells (human intestinal epithelial cell line) was determined by a cell proliferation assay using WST-1 based colorimetric assay. Caco-2 cells ($5*10^3$ cells/well) were plated on a 96-well multiplate and treated with peptides P1, C1 and C2 at different concentrations namely 10, 50 and 100 μM for a period of 24 hours (long term cell viability study). WST-1 reagent (diluted 1:10 in cell culture medium) was added as described in the instruction manual (Roche Diagnostics GMBH, Mannheim, Germany). Following 1-3h incubation at 37 °C with the peptides or controls, absorbance at 450 nm (reference at 690 nm) was measured by a BioTek Synergy Mx plate reader. Percentage of cell viability was calculated based on the absorbance measured relative to that of cells exposed to only culture medium (control group). Sodium dodecyl sulfate (SDS) at 2% was used as a positive control for cell toxicity.

Tested peptides did not show any toxicity on the Caco-2 cells at 10 μM concentration (Figure 7). At a concentration of 50 uM, C2 and P1 did not show any toxicity but C1 showed a cell viability percentage of only 55.2%. At a concentration of 100 μM, C2 showed a cell viability percentage of 77.68% and P1 of 76.7% while C1 has only 34 % of cell viability. Thus, cell toxicity ranking of the peptides was C1 > P1 > C2 **(Figure 7).**

Therefore, peptide of the invention P1 is shown to be non-toxic to cells at concentrations up to at least 50 μM.

These results show that the increase in peptide length decreases cell viability which could be due to a higher residence time of the longer peptides when compared to that of the medium and short sequences and therefore prolonged inhibition of PKC zeta would cause a decrease in cell viability. These result show the effect of the long-term study in comparison to the short-term study (2 hours) performed by Jain et al., 2011, Biochem J., 437(2): 289-299*)* and thus revealed the acute effect of PKC zeta PS peptides.

**Example 6: Effects of peptides of the invention on the redistribution of tight junction proteins occludin and ZO-1**

The effect of peptides of the invention on the tight junction structure of human primary nasal and bronchial epithelial cells were evaluated by confocal microscopy as follows.

Mucilair™ human primary nasal and bronchial epithelial cells (Epithelix Sarl, Geneva, Switzerland) were incubated with 50 μM of peptide of the invention P1 or comparative peptidees C1 and C2 for 2 hours and washed twice with phosphate buffered saline (PBS) (without $Ca^{2+}/Mg^{2+}$) at 37°C. Cells were fixed with methanol/acetone (50:50) for 5 minutes at 20°C, air-dried, and washed with TBST (mixture of tris-buffered saline (TBS) and Polysorbate 20). Cell monolayers were blocked using a solution of 3 % bovine serum albumin (BSA) in PBS for 60 minutes at room temperature and incubated with primary antibody against occludin (Cat: 331588, Invitrogen, Zug, Switzerland); dilution (1: 200)) and ZO-1 (zonula occludens-1 protein) (Cat: 339194, Invitrogen, Zug, Switzerland); dilution (1: 200)) in dilution buffer. Samples were mounted (Vectashield mounting media with DAPI (4',6-diamidino-2-phenylindole); Vector Laboratories) and assessed within the next 24 hours by using a laser-scanning confocal microscope (CLSM; Plan-Apochromat 63/1.40 (oil) DIC objective, Zeiss Axiovert 100M-LSM 510; Carl Zeiss, Oberkochen, Germany). At least 5 individual sites of image capture were chosen randomly in areas of uniform monolayer thickness for each sample. To establish comparable conditions between individual cell monolayers, equivalent images of equal number of horizontal slices (512 * 512 pixels) with the same vertical depth from apical tip to basal membrane between non-stimulated and stimulated mono layers were acquired.

The immunofluorescence images showed that occludin and ZO-1 are co-localized at the intercellular junctions in control cell monolayers. The tested peptides induced redistribution of occludin and ZO-1 from the intercellular junctions into the intracellular compartment

**(Figure 8A and B).**

These results indicate that the PS peptides induce TJ proteins occludin/ZO-1 redistribution and intracellular accumulation in primary human nasal and bronchial epithelial cells. P1 causes desired TJ proteins redistribution that is required for tissue permeabilization useful for molecules delivery.

**Example 7: Effect of the combined treatment comprising peptides of the invention**

The effect of the combination of a peptide of the invention with a protein kinase inhibitor antineoplastic agent, gefitinib (N-(3-Chloro-4-fluorophenyl)-7-methoxy-6-(3-morpholino propoxy) quinazolin-4-amine) known to be a selective inhibitor of the epidermal growth factor receptor's tyrosine kinase domain (EGFR-TK), was investigated in a model of non-small cell lung cancer as follows.

Gefitinib is a cytotxic small molecule first approved in 2003 by the Food and Drug Administration (FDA) as a third-line therapy for the treatment of non-small cell lung cancer (NSCLC), the most common type of lung cancer, and more recently as a first-line treatment (July 2015). Gefitinib has been shown to significantly improve progression free survival (PFS = 7.7-12.9 months) compared to chemotherapies before resistance to treatment appears. In clinic, the usual dose of gefitinib is 250 mg/day while *in vitro* gefitinib has micromolar ($\mu$M) inhibitory concentrations.

HCC827 cells (lung adenocarcinoma) mutated in the tyrosine kinase domain of the EGFR (epidermal growth factor receptor) were used (model OncoCilAir™, wherein Non-Small Cell Lung Cancer (NSCLC) cells were tagged with green fluorescent protein (GFP)). A total of 24 OncoCilAir™ cultures (Mas et al., 2015, J. Biotechnol., 205: 111-119*)* with tumors carrying the EGF receptor ex19:del mutation (noted OCA_EGFR19del) were treated with the gefitinib (or ZD-1839, "Iressa") (Selleckchem (Luzern, Switzerland) in combination or not with peptide P1 for 14 days. The OCA_EGFR19del cultures were prepared from a DMSO (dimethyl sulfoxide) stock diluted in culture medium at two final concentrations, 1 $\mu$M and 5 $\mu$M.

Peptide P1 was administered on the apical side and two types of administration were tried for gefitinib (on the basolateral side of inserts or on the apical side). Peptide P1 was used at a final concentration of 1 $\mu$M in culture medium starting from a 200 $\mu$M stock. P1 was administered to the cultures exactly 5 minutes before the administration of gefitinib.

Tumour morphometry was resolved by fluorescence microscopy using a Zeiss Axiocam microscope platform. Growth curves were based on various images were acquired every 2 days and the area of the green fluorescent protein positive ($GFP^+$) tumour was measured using the Image-Pro Plus Software (MediaCybernetics, Rockville, MD, USA) . For each time point, the ratio of the total area occupied by tumours to the size of the insert was calculated and expressed as percentage of day 0, at the start of the treatment (tumor occupancy). Identical analyses settings, i.e. fixed fluorescence intensity threshold and fixed size threshold (> $900m^2$) were applied to all processed images. Percentage of tumour growth inhibition was calculated using the formula: (1-[tumour occupancy in treatment group/tumour occupancy in control group]$\times$100).

Repeated exposures of the cultures to 1 $\mu$M of P1 alone (6 expositions over the 14 days treatment) had no effect on tumor size, supporting that the peptide of the invention has no anti-tumoral effect *per se* **(Figure 9).**

Fluorescence pictures were recorded for all conditions at 6 different time points during treatment to establish growth curves and quantify the effect of the drugs on tumour progression. The OncoCilAir cultures carrying tumours with the EGFR Ex19:del mutation showed sensitivity to the tyrosine kinase inhibitor gefitinib at both concentrations 5 $\mu$M and 1 $\mu$M when compared to the DMSO control **(Figure 10A and 10B).** Interestingly, gefitinib applied to the apical compartment of the cultures was effective in reducing tumour growth, with even a greater efficiency compared to treatment applied to the basal compartment

**(Figure 10C).**

Combination of 1 $\mu$M of P1 on the apical side with gefitinib on the basal side significantly potentiated the effects of the drug on tumour progression, both at 5 $\mu$M and 1 $\mu$M gefitinib as compared to the gefitinib alone **(Figure 10A and 10B).**

Combination of P1 and 1 $\mu$M gefitinib was administered on the apical side of the cultures imitating that of an aerosol delivery into the lungs. The combination of P1 and gefitinib was significantly more efficacious than the gefitinib alone. Further, the effect of the combination of the small molecule cytotoxic drug gefitinib and P1 was faster than the effect of gefitinib alone since the maximal therapeutic effect was obtained already on the 3rd day **(Figure 10C).** These results support that peptides of the invention can significantly increase the efficacy of drugs, in particular anti-cancer agents by increasing drug penetration and therefore increasing the accessibility of the drugs inside tumours.

This combined therapy approach is of particular interest since in recent gaining body of evidences has shown that TJ proteins have an adhesion-independent role in promoting cancer progression and changes the paradigm that the loss of TJs and cell-cell adhesion are essential for tumor dissemination in the early stages of metastatic cascade

(Leech et al., 2015, Annals of Translational Medicine, 3, 184). More particularly, it has been shown that in NSCLC, increased expression of ZO-1 has been reported and ZO-1 has been attributed to restrict the paracellular permeability between cells *(*Ni et al., 2013, Int. J. Clin. Exp. Patholo., 6: 2887-2895*).* Therefore, the compounds of the invention would be particular interest for use in anti-cancer therapies.

**Example 8: Adjuvant effect of peptides of the invention on antigen specific serum IgG and IgG1 responses**

The effect of peptides of the invention on the efficacy of mucosal vaccines has been investigated by administering a combination of P1 with a soluble protein antigen and induced immunization has been investigated.

6 week-old female C57-BL/6 mice were purchased from Charles River Laboratories (Harlan, France) and hosted under standard conditions following the corresponding guidelines of Animal Ethic Committee. Mice (n = 5) were immunized by intranasal (i.n.) administration under anesthesia on days 0, 14, and 28 with the following immunogens: saline (PBS) as a negative control, ovalbumin (Ova) (in NaCl 0.9% solution) or Ova + P1 Ova/dose (5 μg) (mixture of solution of Ova in NaCl 0.9% and solution of P1 in NaCl 0.9%) as a mixture were administered to each animal group in 12 μL (6 μL per nostril). P1 was used at a concentration of 5 μg/dose. Blood samples were taken one day before the first immunization and one week after the last immunization.

Antigen-specific serum antibodies (IgG total and IgG1) were measured by ELISA (enzyme-linked immunosorbent assay). Briefly, 96-well plates were coated overnight at 4°C with Ova antigen (100 ng) per well. Plates were blocked with 100 μl DPBS (Dulbecco's phosphate-buffered saline) plus 3% BSA (Sigma-Aldrich, Germany) for 2 h at 37°C, washed 4 times with washing buffer, then incubated with 100 μl of serially diluted serum samples (1:50 to 1:819200 for IgG and IgG1) for 1.5 h at 37°C. After washing for 4 times, plates were incubated with 100 μl of a 1:8000 dilution of HRP (horseradish peroxidase)-conjugated antimouse IgG total and IgG1 (Southern Biotech, France) antibodies for 1 h. Plates were washed 4 times, and HRP was quantified by adding 100 μl of TMB (3,3',5,5'-Tetramethylbenzidine) substrate (Pierce Protein Research Products; Rockford, IL). Antibody titers were determined at the midpoint of the optical density-log dilution curves after subtraction of the naive background, and none-responding mice were given an arbitrary titer of 10.

The systemic antigen-specific antibody response was evaluated by quantifying total IgG and IgG1 responses for each group. Groups of BALB/c (an albino, laboratory-bred strain of the House Mouse) mice were i.n. immunized with 5 μg of Ova in the presence or absence of 5 μg of P1 **(Figure 11).** Ova-specific serum IgG (total) and IgG1 responses measured after three immunizations indicated that mice receiving Ova with P1 were significantly higher (P = 0.0079) than those with Ova alone **(Figure 11).** Therefore, peptide P1 significantly improves the immune response to a mucosal vaccine, when co-delivered with such a vaccine. This supports that peptides of the invention may be advantageously used as a mucosal adjuvant for the induction of serum IgG responses, for example through intranasal co-administration with an antigen.

## SEQUENCE LISTING

**SEQ ID NO: 1 (Consensus)**

$$Z\text{- } [Xaa1\ Xaa2\ Xaa3]_{0\text{-}1}\text{-}Xaa_4\ Xaa_5\ R\ Xaa_7\ Xaa_8\ \text{- } [Xaa_9\ Xaa_{10}]_{0\text{-}1}$$

wherein **Z** is a cell penetrating moiety; **Xaa1** and **Xaa2** can be present or absent and, when present, Xaa1 and $Xaa_2$ are independently a positively charged amino acid and **$Xaa_3$** is a non-polar amino acid; **$Xaa_4$** is an amino acid selected from Ala, Ser and Val; **R** is Arginine; **$Xaa_5$** and **$Xaa_8$** are independently a positively charged amino acid; **$Xaa_7$** is a non-polar amino acid; **$Xaa_9$** is a positively charged amino acid and **$Xaa_{10}$** can be present or absent and, when present, $Xaa_{10}$ is a non-polar amino acid.

**SEQ ID NO: 2** (**P1**)
Myr-ARRWR

**SEQ ID NO: 3 (P2)**
Myr-AKRWR

**SEQ ID NO: 4 (comparative peptide C1)**
Myr-SIYRRGARRWRKL

**SEQ ID NO: 5 (comparative peptide C2)**
N-acetyl-ARR

**SEQ ID NO: 6 (comparative peptide C3)**
ARRWR

**Claims**

1. A peptide of 5 to 10 amino acids in total of the following Formula (I):

$$Z\text{- }Z1\text{-}Xaa_4\,Xaa_5\,R\,Xaa_7\,Xaa_8\text{ -}Z2$$

**(I)**

wherein **Z** is a cell penetrating moiety;
**Z1** is an optional peptidic moiety of 1 to 3 amino acids of formula (II):

$$Xaa1\ Xaa2\ Xaa3$$

**(II)**

wherein **Xaa1** and **Xaa$_2$** can be present or absent and, when present, Xaa1 and Xaa$_2$ are independently a positively charged amino acid and **Xaa$_3$** is a non-polar amino acid; **Xaa$_4$** is an amino acid selected from Ala, Ser and Val; **R** is Arginine; **Xaa$_5$** and **Xaa$_8$** are independently a positively charged amino acid; **Xaa$_7$** is a non-polar amino acid**Z2** is an optional peptidic moiety of 1 to 2 amino acids of formula (III):

$$Xaa_9\ Xaa_{10}$$

**(III)**

wherein **Xaa₉** is a positively charged amino acid and **$Xaa_{10}$** can be present or absent and, when present, $Xaa_{10}$ is a non-polar amino acid.

2. A peptide according to claim 1 wherein Z1 is absent.

3. A peptide according to claim 1 or 2 wherein Z2 is absent.

4. A peptide according to any one of the preceding claims wherein $Xaa_4$ is Ala.

5. A peptide according to any one of the preceding claims wherein $Xaa_5$ is Arg.

6. A peptide according to any one of claims 1 to 4 wherein $Xaa_5$ is Lys.

7. A peptide according to any one of the preceding claims wherein $Xaa_8$ is Arg.

8. A peptide according to any one of the preceding claims wherein $Xaa_6$ is Trp.

9. A peptide according to any one of the preceding claims having the following Formula (Ia):

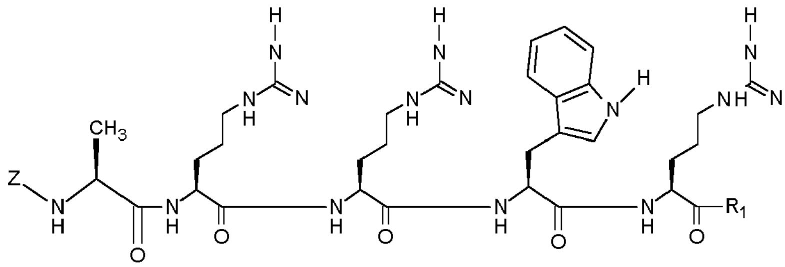

**(Ia)**

Wherein Z is as described in any of the preceding claims and $R_1$ is selected from OH and an amino group, such as $NH_2$.

10. A peptide according to any one of the preceding claims wherein Z is a fatty acid moiety such as a myristoyl group.

11. A peptide according to any one of the preceding claims of SEQ ID NO: 2.

12. A peptide according to any one of the preceding claims for use as a medicament or as a vaccine adjuvant.

13. A pharmaceutical composition comprising at least one peptide according to any one of claims 1 to 11 and a pharmaceutically acceptable carrier, diluent or excipient thereof.

14. A pharmaceutical composition according to claim 13 further comprising an anti-cancer agent.

15. A peptide according to any one of claims 1 to 11 for use in combination with an anti-cancer agent for the prevention and/or treatment of a carcinoma or for use in combination with a vaccine.

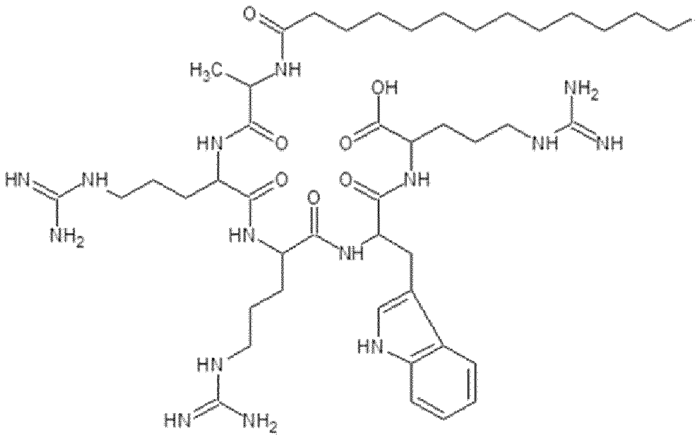

Figure 1

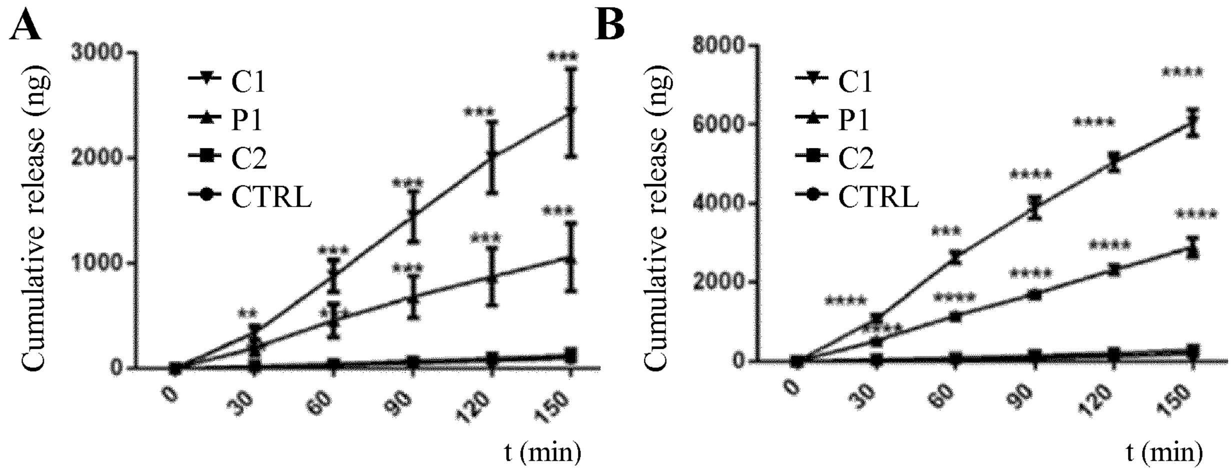


Figure 2

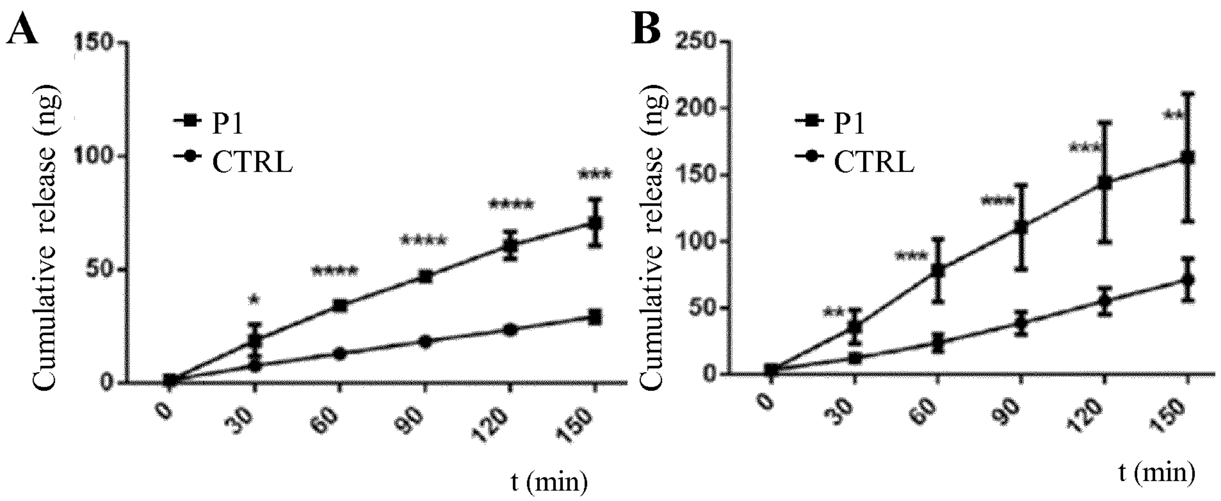


Figure 3

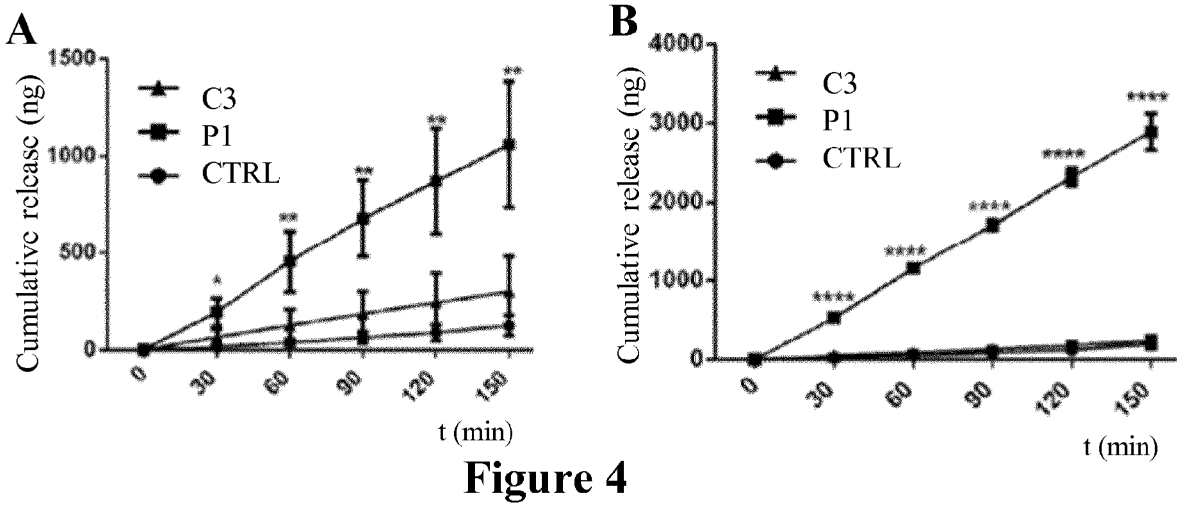


Figure 4

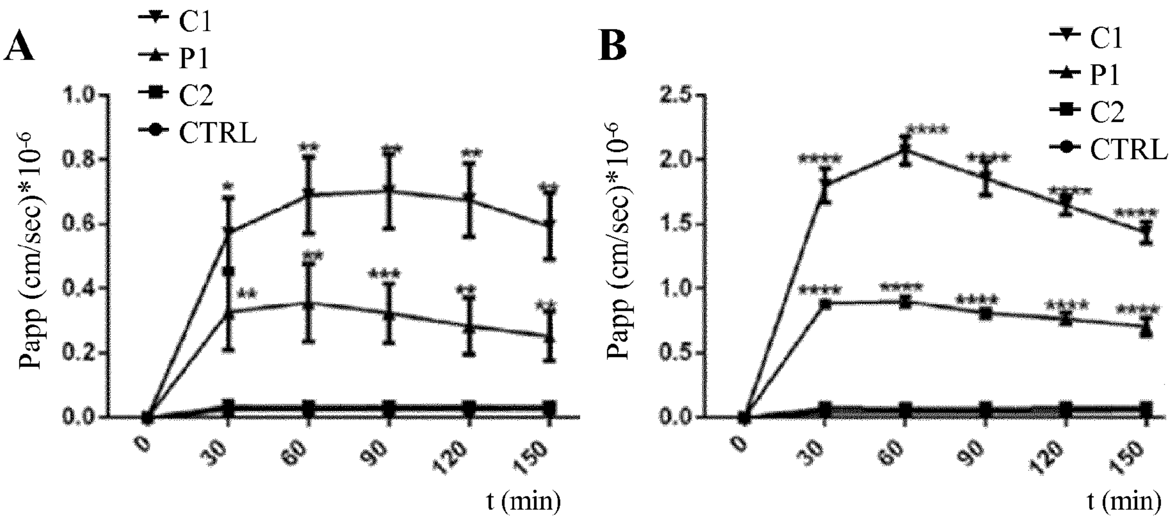


Figure 5

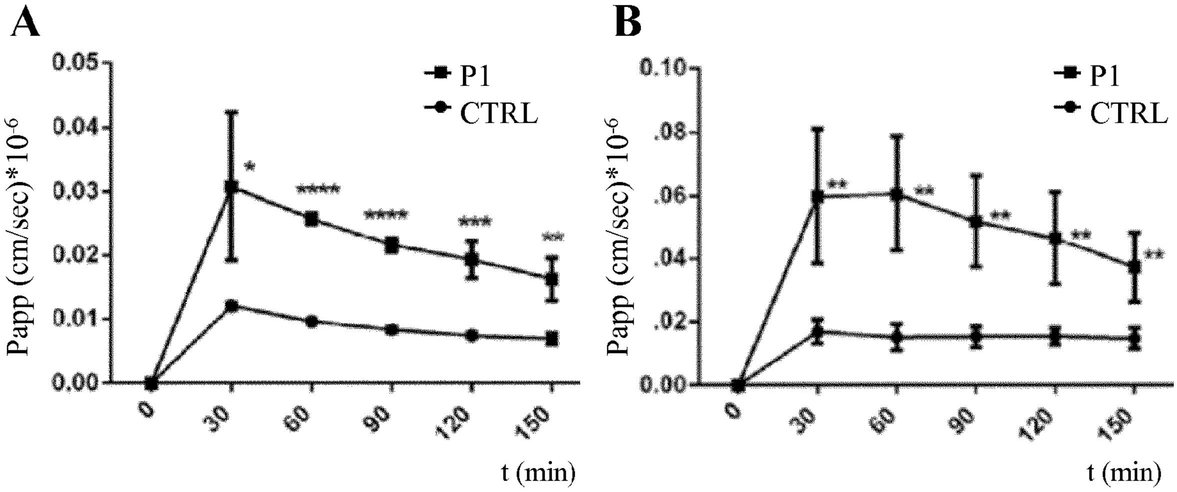


Figure 6

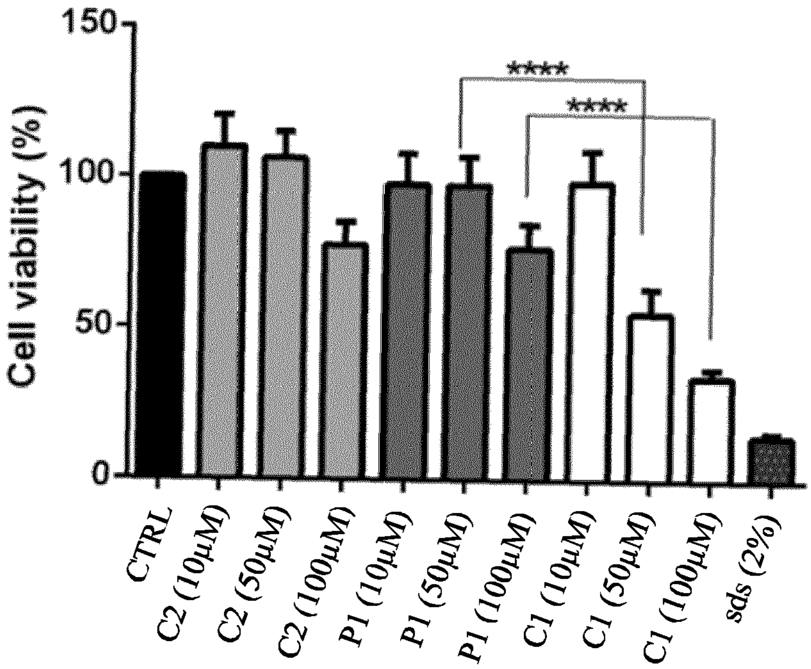


**Figure 7**

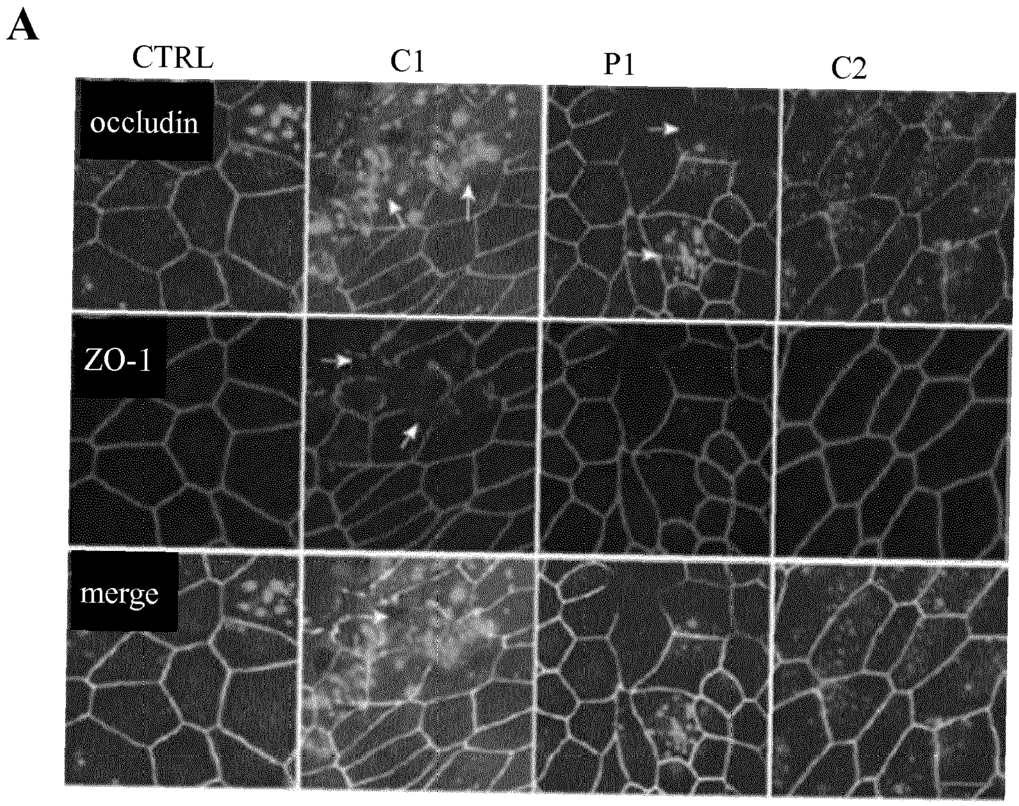


**Figure 8**

**B**

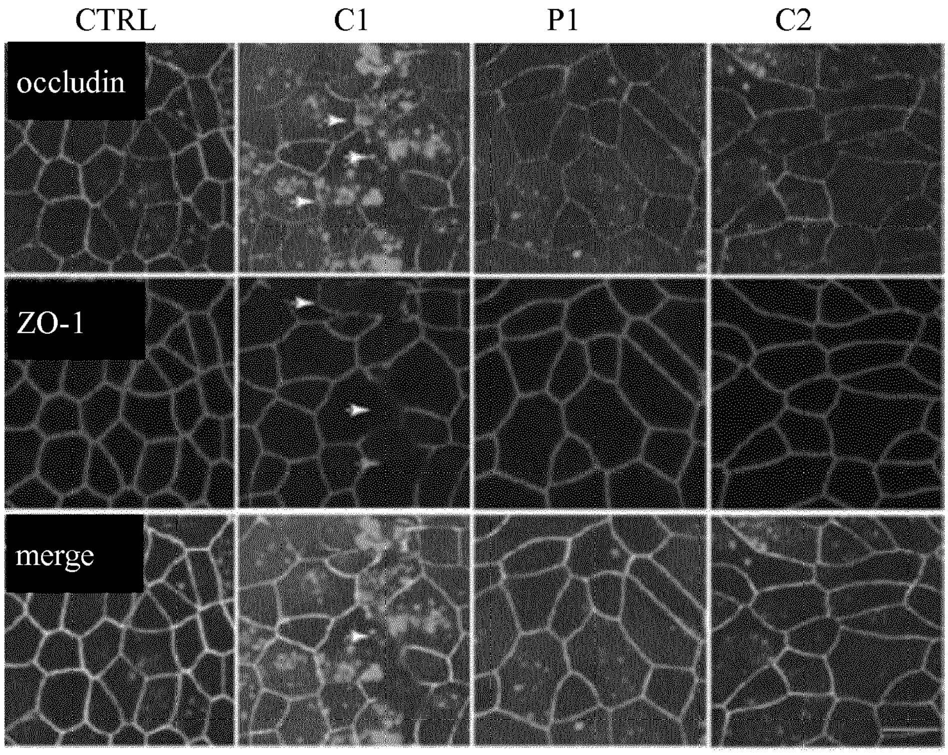


**Figure 8 (continued)**

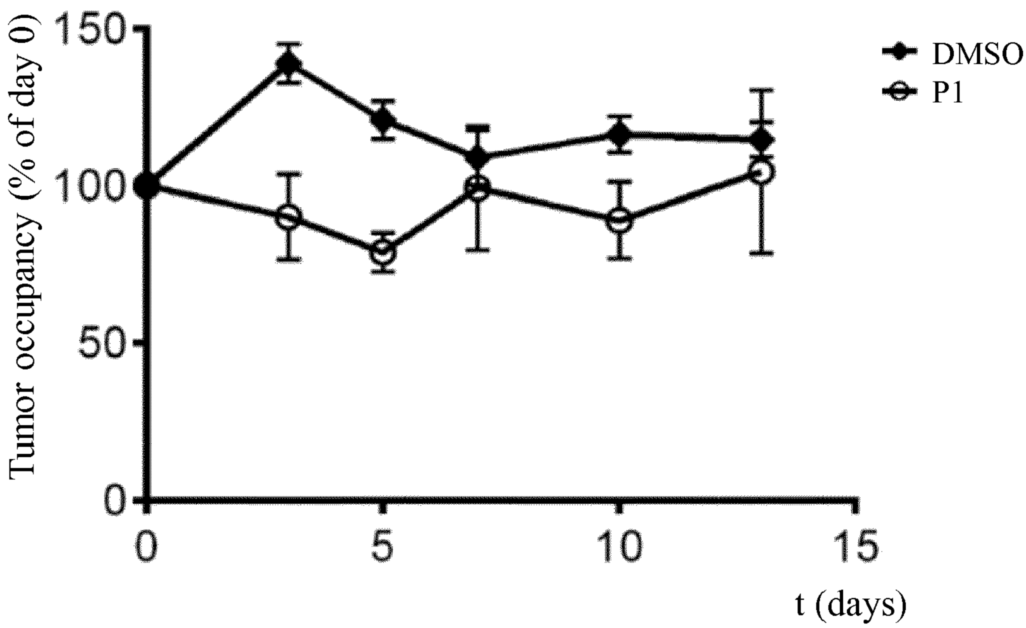


**Figure 9**

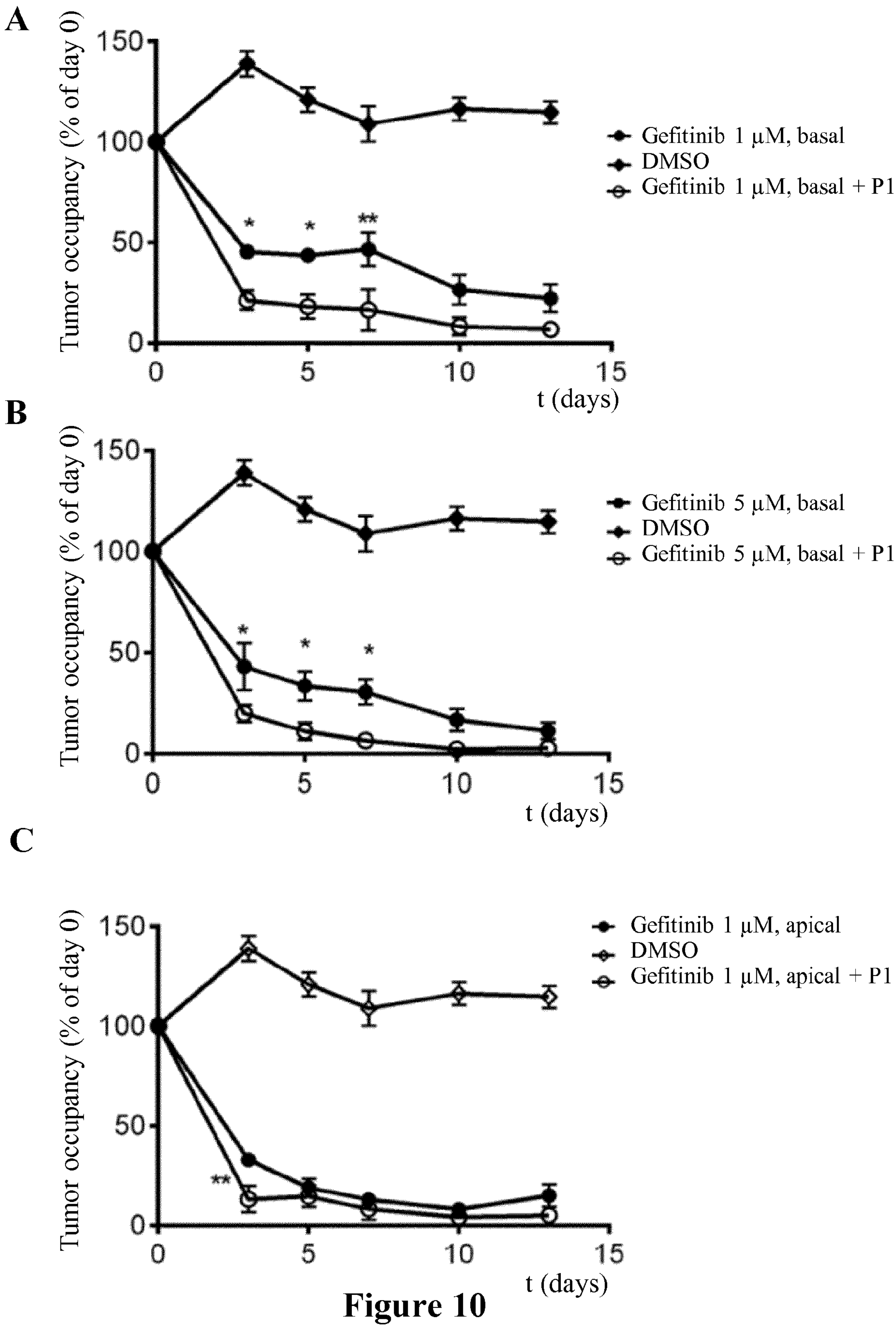
A
Tumor occupancy (% of day 0)
150
100
50
0
0
5
10
15
t (days)
Gefitinib 1 μM, basal
DMSO
Gefitinib 1 μM, basal + P1
*
*
**
B
Tumor occupancy (% of day 0)
t (days)
Gefitinib 5 μM, basal
DMSO
Gefitinib 5 μM, basal + P1
*
*
*
C
Tumor occupancy (% of day 0)
t (days)
Gefitinib 1 μM, apical
DMSO
Gefitinib 1 μM, apical + P1
**

**Figure 10**

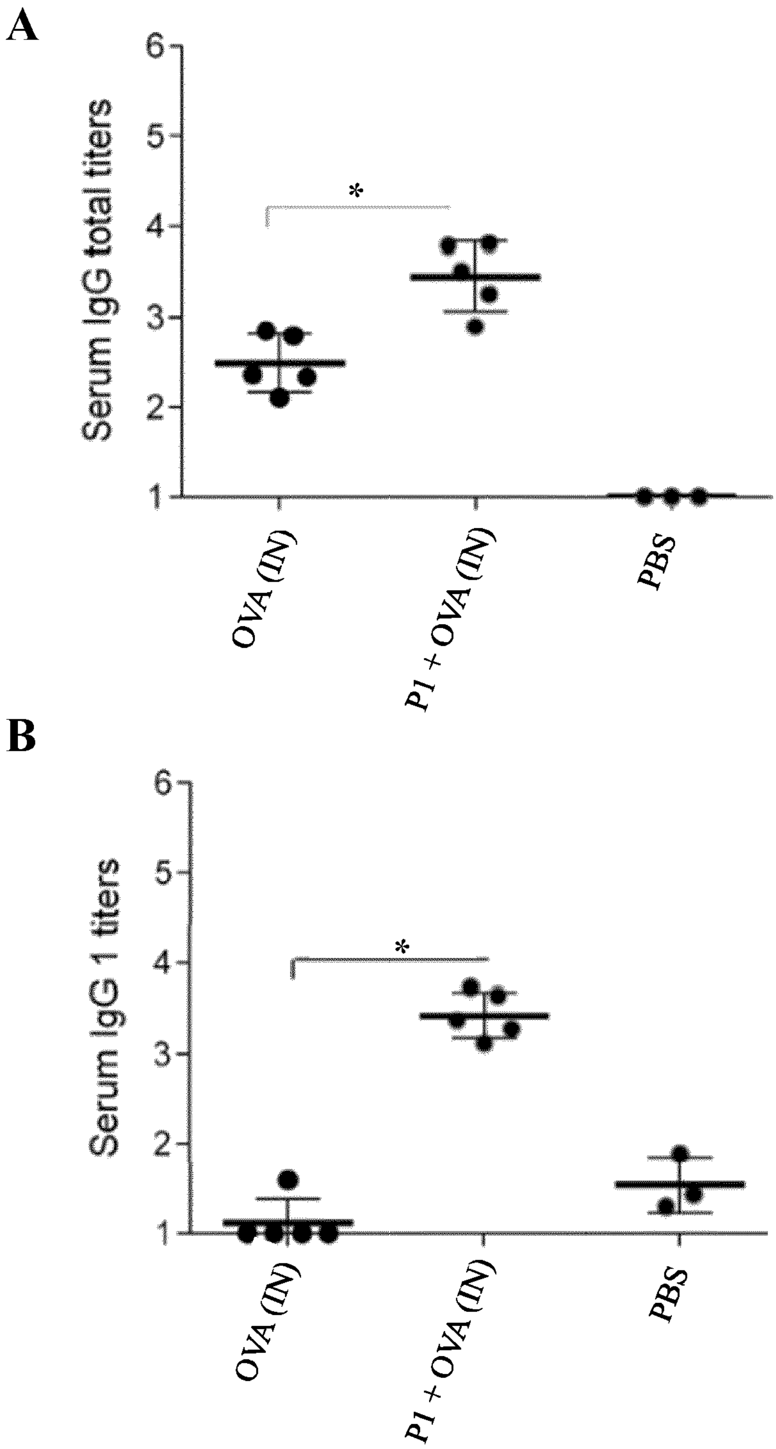
A
Serum IgG total titers
6
5
4
3
2
1
*
OVA (IN)
P1 + OVA (IN)
PBS
B
Serum IgG 1 titers
6
5
4
3
2
1
*
OVA (IN)
P1 + OVA (IN)
PBS

# Figure 11

Europäisches Patentamt
European Patent Office
Office européen des brevets

# EUROPEAN SEARCH REPORT

Application Number
EP 16 20 3107

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X,D | WO 93/20101 A1 (GLAXO SA [ES]; DIAZ MECO CONDE MARIE TERESA [ES]; MOSCAT GUILLEN JORGE) 14 October 1993 (1993-10-14)<br>* p. 3, line 1 - p. 12, line 8, p. 24, Ex. 11, SEQ ID NOs: 3-5, claims 1-24 *<br>----- | 1-15 | INV.<br>C07K7/06<br>C12N9/12 |
| Y | ISABEL DOMINGUEZ ET AL: "Evidence for a Role of Protein Kinase C [zeta] Subspecies in Maturation of Xenopus laevis Oocytes", MOLECULAR AND CELLULAR BIOLOGY, AMERICAN SOCIETY FOR MICROBIOLOGY, WASHINGTON, US, vol. 12, no. 9, 1 September 1992 (1992-09-01), pages 3776-3783, XP001318789, ISSN: 0270-7306<br>* the whole document *<br>----- | 1-15 | |
| Y | BONNET DOMINIQUE ET AL: "SYNTHESIS BY CHEMOSELECTIVE LIGATION AND BIOLOGICAL EVALUATION OF NOVEL CELL-PERMEABLE PKC-ZETA PSEUDOSUBSTRATE LIPOPEPTIDES", JOURNAL OF MEDICINAL CHEMISTRY, AMERICAN CHEMICAL SOCIETY, US, vol. 44, no. 3, 1 February 2001 (2001-02-01), pages 468-471, XP009084311, ISSN: 0022-2623, DOI: 10.1021/JM000920S<br>* the whole document *<br>----- | 1-15 | TECHNICAL FIELDS SEARCHED (IPC)<br>C07K<br>C12N<br>A61K |
| Y | US 2011/245179 A1 (PARK WOO JIN [KR] ET AL) 6 October 2011 (2011-10-06)<br>* p. 5, paragraph [0056] - p. 6, paragraph [0078], SEQ ID NO: 2 *<br>----- | 1-15 | |

The present search report has been drawn up for all claims

1

| Place of search | Date of completion of the search | Examiner |
|---|---|---|
| Munich | 24 March 2017 | R. von Eggelkraut-G. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT ON EUROPEAN PATENT APPLICATION NO. EP 16 20 3107

This annex lists the patent family members relating to the patent documents cited in the above-mentioned European search report. The members are as contained in the European Patent Office EDP file on
The European Patent Office is in no way liable for these particulars which are merely given for the purpose of information.

24-03-2017

| Patent document cited in search report | Publication date | Patent family member(s) | Publication date |
|---|---|---|---|
| WO 9320101 A1 | 14-10-1993 | EP 0592634 A1 | 20-04-1994 |
| | | JP H06508154 A | 14-09-1994 |
| | | WO 9320101 A1 | 14-10-1993 |
| US 2011245179 A1 | 06-10-2011 | KR 20100032351 A | 25-03-2010 |
| | | US 2011245179 A1 | 06-10-2011 |
| | | WO 2010032976 A2 | 25-03-2010 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 9320101 A

### Non-patent literature cited in the description


- **VAN ITALLIE et al.** *Semin. Cell Dev. Biol.,* 2014, vol. 36, 157-165
- **STEINBERG.** *Physiol. Rev.,* 2008, vol. 88, 1341-1378
- **HIRAI et al.** *J. Biochem.,* 2003, vol. 133, 1-7
- **SELBIE et al.** *J. Biol. Chem.,* 1993, vol. 268, 24296-24302
- **HOUSE et al.** *Science,* 1987, vol. 238, 1726-1728
- **KENNEDY.** *Drug Discovery Today,* 1997, vol. 2, 436-444
- **LIPINSKI.** *J. Pharmacol. Toxicol. Methods,* 2000, vol. 44, 235-249
- **DELI.** *Biochim. Biophys. Acta,* 2009, vol. 1788, 892-910
- **YAMAMOTO et al.** *J. Pharm. Pharmacol.,* 1996, vol. 48, 1285-1289
- **SWENSON et al.** *Pharm. Res.,* 1994, vol. 11, 1132-1142
- **BEYER et al.** *Cancer Res.,* 2011, vol. 71, 7080-7090
- **LIPINSKI et al.** *Adv. Drug Deliv. Rev.,* 2001, vol. 46, 3-26
- **LAVIN et al.** *J. Exp. Biol.,* 2007, vol. 210, 2754-2764
- Chitosan-based delivery systems for mucosal vaccination. **BORCHARD et al.** Chitosan-Based Systems for Biopharmaceuticals: Delivery, Targeting and Polymer Therapeutics. John Wiley & Sons, Ltd, 2012, 211-224
- **BONNET et al.** *J. Med. Chem.,* 2001, vol. 44, 468-471
- **SCHWARTZ et al.** *Curr. Opin. Mol. Ther.,* 2000, vol. 2 (2), 162-7
- **LEE et al.** *Methods Mol Biol.,* 2013, vol. 991, 281-92
- **BECHARA et al.** *FEBS Lett.,* 2013, vol. 587 (12), 1693-1702
- **PISA et al.** *J. Pept. Sci.,* 2015, vol. 21 (5), 356-369
- **GUO et al.** *Biomed. Rep.,* 2016, vol. 4 (5), 528-534
- **SVENSEN et al.** *Trends in Pharmacological Sciences,* 2012, vol. 33 (4), 186-192
- **GEURINK et al.** *J. Med. Chem.,* 2013, vol. 56, 1262
- **RAND et al.** *Med. Chem. Commun,* 2012, vol. 3, 1282
- **MEVYN et al.** *Human Vaccines & immunotherapeutics,* 2014, vol. 10 (8), 2175-2187
- **SAE-HAE et al.** *Experimental & Molecular Medicine,* 2014, vol. 46, e85
- **REMINGTON'S.** The Science and Practice of Pharmacy. University of the Sciences in Philadelphia, Lippincott Williams & Wilkins, 2012
- **JAIN et al.** *Biochem J.,* 2011, vol. 437 (2), 289-299
- **HUANG.** *Toxicol. in Vitro,* 2013, vol. 27, 1151-1156
- **SUZUKI.** *J. Cell Sci.,* 2002, vol. 115, 3565-3573
- **WHYTE.** *J. Cell Sci.,* 2010, vol. 123, 3316-3328
- **MAS et al.** *J. Biotechnol.,* 2015, vol. 205, 111-119
- **LEECH et al.** *Annals of Translational Medicine,* 2015, vol. 3, 184
- **NI et al.** *Int. J. Clin. Exp. Patholo.,* 2013, 2887-2895

SEQUENCE LISTING

<110> UNIVERSITE DE GENEVE

<120> PEPTIDIC PROTEIN KINASE C INHIBITORS AND USES THREOF

<130> P2054EP00

<160> 6

<170> PatentIn version 3.3

<210> 1
<211> 8
<212> PRT
<213> Artificial Sequence

<220>
<223> Consensus

<220>
<221> MISC_FEATURE
<222> (1)..(1)
<223> cell penetrating moiety covalently linked to a group of the N-terminal amino acid

<220>
<221> MISC_FEATURE
<222> (2)..(2)
<223> Xaa is an optional peptidic sequence Xaa1 Xaa2 Xaa3 wherein Xaa1 and Xaa2 can be present or absent and, when present, Xaa1 and Xaa2 are independently a positively charged amino acid; Xaa3 is a non-polar amino acid.

<220>
<221> MISC_FEATURE
<222> (3)..(3)
<223> Xaa is an amino acid selected from Ala, Ser and Val.

<220>
<221> MISC_FEATURE
<222> (4)..(4)
<223> Xaa is a positively charged amino acid

<220>
<221> MISC_FEATURE
<222> (6)..(6)
<223> Xaa is a non-polar amino acid

<220>
<221> MISC_FEATURE
<222> (7)..(7)
<223> Xaa is a positively charged amino acid

<220>
<221> MISC_FEATURE
<222> (8)..(8)
<223> Xaa is an optional peptidic moiety [Xaa9 Xaa10] wherein Xaa9 is a positively charged amino acid and Xaa10 can be present or absent and, when present, Xaa10 is a non-polar amino acid.

<400> 1

Xaa Xaa Xaa Xaa Arg Xaa Xaa Xaa
1               5

<210> 2
<211> 5
<212> PRT
<213> Artificial Sequence

<220>
<223> P1

<220>
<221> MISC_FEATURE
<222> (1)..(1)
<223> Myristoyl group covalently linked to an amino group of the N-terminal amino acid

<400> 2

Ala Arg Arg Trp Arg
1               5

<210> 3
<211> 5
<212> PRT
<213> Artificial Sequence

<220>
<223> P2

<220>
<221> MISC_FEATURE
<222> (1)..(1)
<223> Myristoyl group covalently linked to an amino group of the N-terminal amino acid

<400> 3

Ala Lys Arg Trp Arg
1               5

<210> 4
<211> 13
<212> PRT
<213> Artificial Sequence

<220>
<223> comparative peptide C1

<220>
<221> MISC_FEATURE
<222> (1)..(1)
<223> Myristoyl group covalently linked to an amino group of the N-terminal amino acid

<400> 4

```
Ser Ile Tyr Arg Arg Gly Ala Arg Arg Trp Arg Lys Leu
1               5                   10


<210>  5
<211>  3
<212>  PRT
<213>  Artificial Sequence

<220>
<223>  comparative peptide C2


<220>
<221>  MOD_RES
<222>  (1)..(1)
<223>  ACETYLATION

<400>  5

Ala Arg Arg
1


<210>  6
<211>  5
<212>  PRT
<213>  Artificial Sequence

<220>
<223>  comparative peptide C3

<400>  6

Ala Arg Arg Trp Arg
1               5
```